# EUROPEAN PATENT SPECIFICATION

(11) **EP 0 486 653 B1**
(45) Date of publication and mention of the grant of the patent: **22.05.1996**
(21) Application number: 91911054.4
(22) Date of filing: 07.06.1991
(51) Int. Cl.: A61L 2/18

(54) **METHOD FOR PRESERVING CONTACT LENSES IN ONE STEP**
EINSTUFIGES VERFAHREN ZUR KONSERVIERUNG VON KONTAKTLINSEN
PROCEDE POUR CONSERVER LES LENTILLES DE CONTACT EN UNE SEULE ETAPE

(30) Priority: 11.06.1990 ES 9001617
(43) Date of publication of application: 27.05.1992
(73) Proprietor: DISOP, S.A., E-28100 Alcobendas (ES)
(72) Inventor: ARRANZ AGUIRRE, Juan, E-28100 Alcobendas (ES)
(74) Representative: Carpintero Lopez, Francisco
(86) International application number: ES9100033
(87) International publication number: WO9119517

(56) References cited:
- EP-A- 0 209 071

## Description

### OBJECT OF THE INVENTION

The present invention relates to a single-step process for preserving contact lenses. This process allows the lenses to be disinfected and after some time neutralised, thereby to be placed directly on the eyes without having to be rinsed.

### BACKGROUND OF THE INVENTION

In the matter of the problems involved in disinfecting contact lenses, it is a fact that sterilization by means of oxidising systems such as hydrogen peroxide is most efficient and safest. This safety is supplemented with a faster disinfection, comparing the time taken by disinfectant solutions containing preservatives (thimerosal, chlorhexidine, ...), usually between 6 and 8 hours, with the 15 minutes required for 3% hydrogen peroxide solutions.

Once disinfection with the hydrogen peroxide solution is over, the contact lenses must be immersed in a neutralising saline solution capable of decomposing (neutralising) the peroxide remaining in the contact lenses. To such end, most manufacturers use catalase enzyme in the neutralizer solution, for the contact lenses to be placed upon the eye within not more than 15 minutes.

These conventional systems comprising a disinfectant hydrogen peroxide solution and a neutralizer solution, are referred to as two-step systems for the user must proceed to replace the disinfectant solution with the neutralizer solution.

Recently, Allergan replaced the neutralizer solution with a tablet carrying out the same function, i.e., once the lenses are disinfected with the peroxide solution, a tablet is incorporated to the same solution which can deactivate all the hydrogen peroxide and convert the solution into a medium compatible with the eye. This new system could also be referred to as a two-step system, because of the user's involvement.

Ciba Geigy has launched a system to the market whereby the hydrogen peroxide disinfectant solution is poured into a container having a platinum black coated ring that can deactivate the peroxide. This system, as opposed to former ones, involves a single step, for the user need not be involved in the treatment.

However, the platinum ring starts to decompose the peroxide immediately, without waiting for a sufficient amount of time to guarantee disinfection, and is therefore being heavily criticized.

The single-step system is also found in (European Patent application no. 86109361.5), EP-A-0 209 071 for instance, where a system similar to ours is mentioned, among other inventions, though none of the claims mentions the fact that the contact lens can actually be placed directly on the eye after being treated. In fact, the coating mentioned for an acid medium is a dimethylaminomethacrylate polymer and neutral methacrylate esters which we have found to be extremely irritating for the eye, and the lens must therefore be thoroughly rinsed. As to the neutral medium, reference is made to coating agents that, either because of their irritability or their insolubility, mean that the lens must be rinsed before being used. In both cases, subsequent rinsing of the lens is a disadvantage for single-step systems.

### ADVANTAGES OF THE INVENTION

Our invention places particular emphasis on the absence of the said rinsing as being a clear advantage and improvement in respect of the said patent. Thus, clinical surveys involving a group of twenty people for a test period of two months show that the system being described in this invention is wholly compatible with the human eye.

Our system also involves only one step since the user need at no time be involved while the contact lenses are being treated. The nature of the tablet allows the peroxide disinfection and neutralization processes not to be simultaneous, but rather neutralization begins once disinfection is over. This represents a clear advantage over the platinum ring system.

### DESCRIPTION OF THE INVENTION

This invention relates to a method for disinfecting, moistening, and neutralizing contact lenses. In particular, it comprises simultaneously incorporating a hydrogen peroxide solution (disinfectant solution) and a tablet containing an active peroxide enzyme (catalase) to the case housing the contact lenses. Within not more than two hours, three things are achieved: the contact lenses are disinfected, moistened and the medium is transformed into a saline solution that is wholly compatible with the eye.

By means of the special coating we provide on the surface of the neutralizing tablet, once the lens is disinfected, the tablet begins its neutralizing, moistening action, lubrifying the contact lens and converting the solution into an isotonic saline solution, with a pH of 7.2-7.4 requiring no rinsing and allowing the lenses to be placed directly on the eyes.

The hydrogen peroxide disinfectant solution moreover contains a stabilizer agent preventing decomposition of the peroxide (for instance, acetylphenetidine) and a buffer (for instance, monosodium phosphate).

The tablet also contains the peroxide neutralizer ingredient, sodium chloride, in a proportion that converts 10 ml of the disinfecting solution into an isotonic solution and disodium phosphate in a quantity sufficient to convert the pH of 3.0 of the said 10 ml of peroxide disinfectant solution into a pH of 7.2-7.4.

The coating of the tablet is a polymer soluble in the medium, polyvinylpyrrolidone, which is added in a quantity sufficient to retard neutralization of the peroxide for some fifteen minutes. The coating can be provided by simple methods such as spraying the polymer on a coating container provided with suction means; or using methods that are easier to control such as fluidized bed, Accela cota, Pellegrini. Polymer spraying requires the use of solvents such as sterile water, ethanol, isopropyl alcohol, dichloromethane, acetone.

The overall process could be described as follows:
The case containing the contact lenses is provided with 10 ml of disinfectant solution and a neutralizer tablet. For the first 10 minutes the solution disinfects the lenses and the tablet's coating dissolves slowly. After 10 or 15 minutes, slight neutralization begins, to reach its peak after 15-17 minutes. Then, and for a further 10-25 minutes, total neutralization is achieved. After 60 minutes, the tablet will have dissolved totally and the lenses will be ready to be used.

### COMPOSITION OF THE TABLET

The following intervals are appropriate for the peroxide disinfectant solution and the neutralizer tablet:

### DISINFECTANT SOLUTION

- 2.8 - 3.5 % w/v: Hydrogen Peroxide
- 0.2 - 0.3 % w/v: Monosodium phosphate x 2 H₂O
- 0.008 - 0.015 % w/v: Acetylphenetidine
Adjustment to pH of 2.8 - 3.2 with 37 % hydrochloric acid

### NEUTRALIZER TABLET (for 10 ml of disinfectant solution)

- 50 - 70 mg: NaCl
- 60 - 100 mg: Disodium phosphate x 2 H₂O
- 2080 - 5400 UI: Lyophilized catalase
- 60 - 110 mg: Polyvinylpyrrolidone (Coating polymer)
Below are some examples of compositions to put the invention into practice:

### EXAMPLE 1

### DISINFECTANT SOLUTION

- 3.2 % w/v: Hydrogen Peroxide
- 0.20 w/v: Monosodium phosphate x 2 H₂O
- 0.013 % w/v: Acetylphenetidine
Adjustment to pH of 3.0 with 37 % hydrochloric acid

### NEUTRALIZER TABLET (for 10 ml of disinfectant solution)

- 58 mg: NaCl
- 72 mg: Disodium phosphate x 2 H₂O
- 4000 UI: Lyophilized catalase
- 90 mg: Polyvinylpyrrolidone K-30 (Coating polymer)

### EXAMPLE 2

### DISINFECTANT SOLUTION

- 3.0 % w/v: Hydrogen Peroxide
- 0.22 w/v: Monosodium phosphate x 2 H₂O
- 0.009 % w/v: Acetylphenetidine
Adjustment to pH of 3.0 with 37 % hydrochloric acid

### NEUTRALIZER TABLET (for 10 ml of disinfectant solution)

- 68 mg: NaCl
- 75 mg: Disodium phosphate x 2 H₂O
- 2700 UI: Lyophilized catalase
- 100 mg: Polyvinylpyrrolidone K-30 (Coating polymer)
When contact lenses were disinfected with these two formulae, the user was able to place them directly on the eye without experiencing any discomfort whatsoever and even felt more comfortable than before when using other peroxide disinfecting systems. A total of 200 tests were made to different people.

It must be borne in mind that these examples are set forth to illustrate and not restrict the present invention, and we now consider the nature of the invention to have been fully described.

## Claims

1. A single-step process for preserving contact lenses, where the lenses are first disinfected and then neutralized after a certain time, in order that they may be directly placed upon the eyes without having to be rinsed, which process involves immersing the contact lens in a system comprising a peroxide-based disinfectant solution and a component to neutralize the same, characterised in that the said neutralizer component is provided as a tablet fully externally coated with a layer of polyvinylpyrrolidone, such tablet with the following composition:
40 - 100 mg Sodium chloride
60 - 160 mg Disodium phosphate 2-hydrate
1080 - 27000 UI Lyophilized catalase
and the quantity of the polyvinylpyrrolidone being between 20 and 50 % in accordance with the total weight of the neutralizer tablet, such polyvinylpyrrolidone layer acts to retard the neutralization of the disinfectant's peroxide content, and the said tablet must therefore be dissolved in some 10 ml of disinfectant solution.

2. A single-step process for preserving contact lenses, as in claim 1, characterized in that the total immersion time of the contact lenses is at least 15 minutes.

3. A single-step process for preserving contact lenses, as in any the previous claims, characterized in that the neutralizer tablet is coated with a polyvinylpyrrolidone layer which acts to retard the neutralization of the peroxide, comprising the following solution:
55 - 65 mg Sodium chloride
65 - 75 mg Disodium phosphate 2-hydrate
2700 - 5400 UI Lyophilized catalase
such tablet being added to some 10 ml of the disinfectant solution, the total immersion time being of roughly 45 minutes.

4. A single-step process for preserving contact lenses, as in claim 3, characterized in that the polyvinylpyrrolidone is polyvinylpyrrolidone K-30.

5. A single-step process for preserving contact lenses, as in claim 4, characterized in that the amount of polyvinylpyrrolidone K-30 used in the tablet coating is between 65 and 75 mg.

6. A single-step process for preserving contact lenses, as in claims 1 to 5, characterized in that the polyvinylpyrrolidone coating in the neutralizer tablet is applied by spraying a polymer film in the pelletting container.

7. A single-step process for preserving contact lenses, as in claims 1 to 5, characterized in that the polyvinylpyrrolidone coating in the neutralizer tablet is applied on a fluidized bed coating device.

## Patentansprüche

1. Einstufiges Verfahren zur Konservierung von Kontaktlinsen, bei dem die Kontaktlinsen im Prinzip so desinfiziert werden, daß sie ohne weitere Spülung unmittelbar auf das Auge gelegt werden können, und welches darin besteht, die Kontaktlinsen in eine Desinfektionslösung zu legen, welche sich aus Peroxid und einem dasselbe neutralisierenden Mittel zusammensetzt, dadurch gekennzeichnet, daß das Neutralisierungsmittel in Form einer außen vollständig mit Polyvinylpyrrolidon beschichteten Tablette folgender Zusammensetzung hinzugegeben wird,
40 - 100 mg Natriumchlorid
60 - 160 mg 2-Hydrat-Dinatriumphosphat
1080 - 2700 UI lyophilisierte Katalase
und die Menge des Polyvinylpyrrolidons zwischen 20% und 50% liegt, jeweils in Abhängigkeit vom Gesamtgewicht der Neutralisierungstablette, wobei die Polyvinylpyrrolidonbeschichtung die Neutralisierung des Peroxidgehalts des Desinfektionsmittels verzögert und die Tablette deshalb in etwa 10 ml der Desinfektionslösung gelöst werden muß.

2. Einstufiges Verfahren zur Konservierung von Kontaktlinsen, nach Anspruch 1 dadurch gekennzeichnet, daß die Kontaktlinsen während eines Zeitraums von mindestens 15 Minuten eingetaucht werden.

3. Einstufiges Verfahren zur Konservierung von Kontaktlinsen, nach den vorstehenden Ansprüchen dadurch gekennzeichnet, daß die Neutralisierungstablette mit Polyvinylpyrrolidon beschichtet wird, das die Neutralisierung des Peroxids verzögert und folgende Zusammensetzung hat:
55 - 65 mg Natriumchlorid
65 - 75 mg 2-Hydrat-Dinatriumphosphat
2700 - 5400 UI lyophilisierte Katalase
und einer Menge von etwa 10 ml der Desinfektionslösung zugegeben wird, wobei die Behandlungszeit insgesamt annähernd 45 Minuten beträgt.

4. Einstufiges Verfahren zur Konservierung von Kontaktlinsen, nach Anspruch 3 dadurch gekennzeichnet, daß es sich bei dem Polyvinylpyrrolidon um Polyvinylpyrrolidon K-30 handelt.

5. Einstufiges Verfahren zur Konservierung von Kontaktlinsen, dadurch gekennzeichnet, daß die in Tablettenbeschichtung verwendete Menge an Polyvinylpyrrolidon K-30 65 - 75 mg beträgt.

6. Einstufiges Verfahren zur Konservierung von Kontaktlinsen, nach Anspruch 1 dadurch gekennzeichnet, daß die für die Neutralisierungstablette verwendete Polyvinylprrolidonbeschichtung durch Zerstäuben eines Polymerfilms im Dragierbehälter aufgebracht wird.

7. Einstufiges Verfahren zur Konservierung von Kontaktlinsen, nach Anspruch 1 dadurch gekennzeichnet, daß die für die Neutralisierungstablette verwendete Polyvinylprrolidonbeschichtung im Flußbett aufgebracht wird.

## Revendications

1. Un procédé pour la conservation de verres de contact en un seul pas, dans lequel initialement les verres sont désinfectés, de telle sorte qu'il est possible de les appliquer directement à l'oeil sans qu'il ne soit nécessaire de les rincer; et dont le processus consiste à submerger le verre de contact dans un système constitué par une solution désinfectante à base de peroxyde et un composant qui neutralise ce dernier, caractérisé par le fait que ce composant de neutralisation est fourni sous forme de comprimé tout à fait recouvert à l'extérieur par une couche de polyvinyle-pyrrolidone, ce comprimé ayant la composition suivante:
40 - 100 mg Chlorure de sodium
60 - 160 mg Phosphate disodique 2-hydrate
1080 - 27 000 UI Catalase lyophilisée
et la quantité de la polyvinyle-pyrrolidone est comprise entre 20% et 50% selon le poids total du comprimé de neutralisation, cette couverture de polyvinylepyrrolidone servant à retarder la neutralisation du contenu de peroxyde du désinfectant, et, par conséquent, ce comprimé doit être dissous en 10 ml, environ, de solution désinfectante.

2. Un procédé pour la conservation de verres de contact en un seul pas, selon la première revendication, caractérisé par le fait que le temps total d'immersion des verres de contact soit d'au moins 15 minutes.

3. Un procédé pour la conservation de verres de contact en un seul pas, selon les revendications précédentes, caractérisé par le fait que le comprimé de neutralisation soit recouvert à l'extérieur par une couche de polyvinyle-pyrrolidone qui agit pour retarder la neutralisation du peroxyde, qui comprend la solution suivante:
55 - 65 mg Chlorure de sodium
65 - 75 mg Phosphate monosodique 2-hydrate
2700 - 5400 UI Catalase lyophilisée
ce comprimé étant rajouté à environ 10 ml de la solution désinfectante, le temps total d'immersion étant d'à peu près 45 minutes.

4. Un procédé pour la conservation de verres de contact en un seul pas, selon la 3ème revendication, caractérisé par le fait que la polyvinyle-pyrrolidone soit la polyvinyle-pyrrolidone K-30.

5. Un procédé pour la conservation de verres de contact en un seul pas, caractérisé par le fait que la quantité de polyvinyle-pyrrolidone K-30 employée dans la couverture du comprimé soit de 65 à 75 mg.

6. Un procédé pour la conservation de verres de contact en un seul pas, selon la 1ère revendication, caractérisé par le fait que la couverture de polyvinylepyrrolidone employée dans le comprimé de neutralisation soit appliquée à l'aide de la pulvérisation d'une pellicule de polymère dans le récipient **à dragées**.

7. Un procédé pour la conservation de verres de contact en un seul pas, selon la 1ère revendication, caractérisé par le fait que la couverture de polyvinylepyrrolidone employée dans le comprimé de neutralisation soit appliquée sur un dispositif de couverture par lit fluidifié.
